(19) **Europäisches Patentamt / European Patent Office / Office européen des brevets**

(11) **EP 4 224 160 A1**

(12) **EUROPEAN PATENT APPLICATION**
published in accordance with Art. 153(4) EPC

(43) Date of publication:
09.08.2023 Bulletin 2023/32

(21) Application number: 21875464.6

(22) Date of filing: 24.09.2021

(51) International Patent Classification (IPC):
$G01N\ 33/30$ (2006.01)  $G01N\ 27/00$ (2006.01)
$G01N\ 27/02$ (2006.01)  $G01N\ 27/22$ (2006.01)

(52) Cooperative Patent Classification (CPC):
G01N 27/00; G01N 27/02; G01N 27/22;
G01N 33/30

(86) International application number:
PCT/JP2021/035203

(87) International publication number:
WO 2022/071162 (07.04.2022 Gazette 2022/14)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 29.09.2020 JP 2020163961
25.08.2021 JP 2021137562

(71) Applicant: NSK Ltd.
Tokyo 141-8560 (JP)

(72) Inventors:
• IWASE, Shunsuke
  Fujisawa-shi, Kanagawa 251-8501 (JP)
• MARUYAMA, Taisuke
  Fujisawa-shi, Kanagawa 251-8501 (JP)

(74) Representative: Grünecker Patent- und
Rechtsanwälte
PartG mbB
Leopoldstraße 4
80802 München (DE)

(54) **STATE DIAGNOSIS METHOD, STATE DIAGNOSIS DEVICE, AND PROGRAM**

(57) A condition diagnosis method includes: a measurement step of measuring relative dielectric constant of a lubricant by applying a voltage to the lubricant while changing a frequency from an AC power source; a derivation step of deriving parameters indicating electrical properties of the lubricant by applying the relative dielectric constant measured in the measurement step to a theoretical formula; and a diagnosis step of diagnosing a condition of the lubricant using the parameters.

*FIG.8*

```
                START
                  │
S801 ──  INSTRUCT INPUT OF ω AND V
                  │
S802 ──  ACQUIRE Z AND θ
                  │
S803 ──  DERIVE RELATIVE DIELECTRIC CONSTANT AND
         RELATIVE DIELECTRIC LOSS FACTOR
         CORRESPONDING TO EACH FREQUENCY
                  │
S804 ──  PERFORM FITTING TO THEORETICAL FORMULA
                  │
S805 ──  DERIVE PARAMETERS FROM
         THEORETICAL FORMULA
                  │
S806 ──  PERFORM CONDITION DIAGNOSIS BASED
         ON PARAMETERS
                  │
S807 ──  NOTIFY USER OF DIAGNOSIS RESULT
                  │
                 END
```

EP 4 224 160 A1

**Description**

TECHNICAL FIELD

The present invention relates to a condition diagnosis method, a condition diagnosis device, and a program.

BACKGROUND ART

Conventionally, when diagnosing the condition of materials, methods such as physical property analysis (for example, apparent viscosity), chemical component analysis (for example, deterioration degree), and structural analysis using a microscope are used. Such methods generally require complicated operations. Moreover, it is difficult to perform the above-described methods non-destructively on the target, and in many cases, the target to be diagnosed is discarded.
On the other hand, there is a method of performing impedance analysis using an AC power source. Impedance analysis can be used to make it possible to derive the electrical properties of the material.
For example, Patent Literature 1 discloses a method of using blood as a target to be analyzed and measuring the dielectric constant of the blood while changing the frequency to measure damage to blood cells. Further, Patent Literature 2 discloses a method of evaluating changes in the composition of an extraction solvent based on frequency characteristics of relative dielectric constant and relative dielectric loss factor.

CITATION LIST

PATENT LITERATURE

Patent Literature 1: JP-A-2008-215901
Patent Literature 2: JP-A-H7-239316

SUMMARY OF INVENTION

TECHNICAL PROBLEM

Materials used in actual devices include, for example, lubricants. Understanding the condition of the lubricant is extremely useful in preventing damage or the like to devices that use the lubricant. On the other hand, it is difficult to observe the target such as a lubricant by taking the target out of the device in a non-destructive manner.
In view of the above problems, an object of the present invention is to provide a method for easily diagnosing the condition of a lubricant without destroying the target to be diagnosed.

SOLUTION TO PROBLEM

In order to solve the above problems, the present invention has the following configuration. In other words, there is provided a condition diagnosis method including: a measurement step of measuring relative dielectric constant of a lubricant by applying a voltage to the lubricant while changing a frequency from an AC power source; a derivation step of deriving parameters indicating electrical properties of the lubricant by applying the relative dielectric constant measured in the measurement step to a theoretical formula; and a diagnosis step of diagnosing a condition of the lubricant using the parameters.
Moreover, another aspect of the present invention has the following structure. In other words, there is provided a condition diagnosis device including: a measurement unit for measuring relative dielectric constant of a lubricant by applying a voltage to the lubricant while changing a frequency from an AC power source; a derivation unit for deriving parameters indicating electrical properties of the lubricant by applying the relative dielectric constant measured in the measurement unit to a theoretical formula; and a diagnosis unit for diagnosing a condition of the lubricant using the parameters.
Moreover, another aspect of the present invention has the following structure. In other words, there is provided a program for causing a computer to execute a measurement step of measuring relative dielectric constant of a lubricant by applying a voltage to the lubricant while changing a frequency from an AC power source, a derivation step of deriving parameters indicating electrical properties of the lubricant by applying the relative dielectric constant measured in the measurement step to a theoretical formula, and a diagnosis step of diagnosing a condition of the lubricant using the parameters.

ADVANTAGEOUS EFFECTS OF INVENTION

According to the present invention, it is possible to easily diagnose the condition of a lubricant without destroying the target to be diagnosed.

BRIEF DESCRIPTION OF DRAWINGS

Fig. 1 is a conceptual diagram showing a configuration of a lubricant which is a target to be diagnosed and an AC power source according to the present invention.
Fig. 2 is a diagram for explaining an equivalent circuit composed of a lubricant which is a target to be diagnosed and an AC power source according to the present invention.
Fig. 3 is a schematic configuration diagram showing an example of a device configuration according to the present invention.
Figs. 4A and 4B are diagrams for explaining a relationship between frequency, and relative dielectric constant and relative dielectric loss factor.
Figs. 5A and 5B are diagrams for explaining derivation of parameters by fitting to a theoretical formula (Debye type).
Figs. 6A and 6B are diagrams for explaining derivation of parameters by fitting to a theoretical formula (Cole-Cole type).
Figs. 7A and 7B are diagrams for explaining derivation of parameters by fitting to a theoretical formula (Cole-Cole improved type).
Fig. 8 is a flowchart of condition diagnosis processing according to one embodiment of the present invention.
Figs. 9A and 9B are diagrams for explaining a relationship between a condition of the lubricant and parameters.
Fig. 10 is a diagram for explaining a relationship between a condition of the lubricant and parameters.
Fig. 11 is a diagram for explaining a relationship between a thickener and relaxation strength.
Figs. 12A and 12B are diagrams for explaining a roll state of the thickener.
Figs. 13A and 13B are diagrams for explaining a relationship between frequency and parameters according to a fiber state of thickener.
Fig. 14 is a diagram for explaining a relationship between frequency and parameters according to a fiber state of thickener.
Figs. 15A and 15B are diagrams for explaining a relationship between deterioration of base oil and relative dielectric constant.
Figs. 16A and 16B are diagrams for explaining a relationship between deterioration of base oil and relative dielectric loss factor.
Figs. 17A and 17B are diagrams for explaining a relationship between deterioration of grease and parameters.
Figs. 18A and 18B are diagrams for explaining a relationship between a water content in grease and parameters.
Fig. 19 is a diagram for explaining a relationship between a water content in grease and average dielectric constant.
Fig. 20 is a diagram for explaining a relationship between an iron powder amount in grease and relative dielectric loss factor.

DESCRIPTION OF EMBODIMENTS

Hereinafter, embodiments of the present invention will be described below with reference to the drawings. In addition, the embodiment described below is one embodiment for describing the present invention, and is not intended to be construed as limiting the present invention. Not all configurations are essential configurations for solving the problems of the present invention. Moreover, in each drawing, the same component is indicated by the same reference number to indicate the correspondence relationship.

[Target to be diagnosed]

In the present embodiment, a lubricant used for lubricating components will be described as an example of a target to be diagnosed. The lubricant used here is grease having a characteristic of causing dielectric relaxation. More specifically, lithium 12-hydroxystearate grease and the like can be targeted. Grease generally is composed of a base oil, a thickener, and additives. Although the details will be described later, the values of the parameters indicating the electrical properties will fluctuate depending on the configuration or condition of the grease.

In the present embodiment, assuming that the grease is in a bulk state, parameters indicating electrical properties corresponding to the internal condition are derived, and the condition of the grease is diagnosed using the parameters.

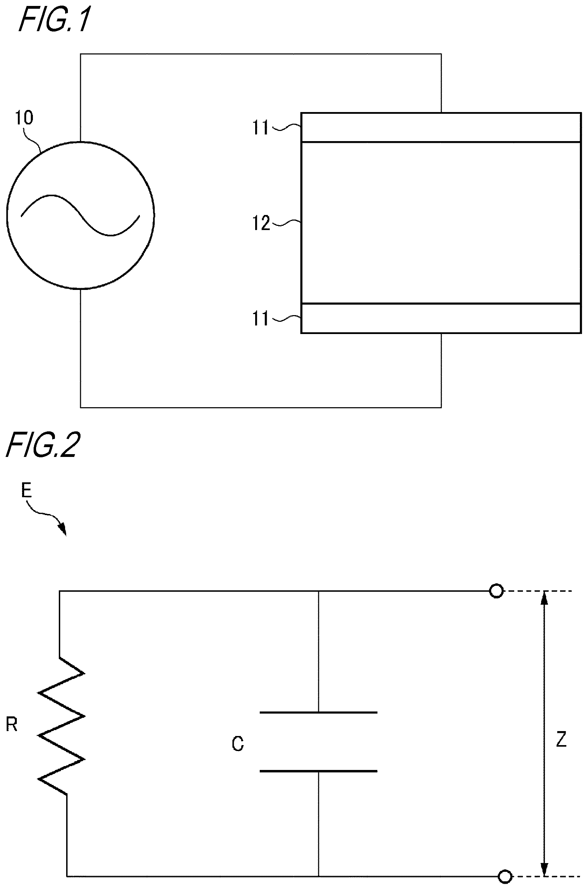

Fig. 1 is a conceptual diagram showing the configuration of a lubricant (here, grease) and an AC power source when evaluating (measuring) the electrical properties of the lubricant according to the present embodiment. Electric power is supplied from an AC power source 10 to grease 12 filled between electrodes 11. In addition, the distance between the electrodes 11 here may be configured on the order of mm, for example.

Fig. 2 is a diagram showing an electrically equivalent electric circuit around the grease 12 shown in Fig. 1. An electric circuit E has a configuration in which a capacitor C composed of the grease 12 and a resistance R caused by peripheral elements are connected in parallel. In addition, the impedance of the electric circuit E is indicated by Z. Here, the AC voltage V applied to the electric circuit E, the current I flowing through the electric circuit E, and the complex impedance Z of the entire electric circuit E are expressed by the following Equations (1) to (3).

$$V = |V|e \times p(j\omega t) \ldots (1)$$

$$I = |I|e \times p(j\omega t - j\theta) \ldots (2)$$

$$Z = V/I = |V/I|e \times p(j\theta) = |Z|e \times p(j\theta) \ldots (3)$$

j: Imaginary number
$\omega$: Angular frequency of voltage
t: Time
$\theta$: Phase angle (phase shift between voltage and current)

[Device configuration]

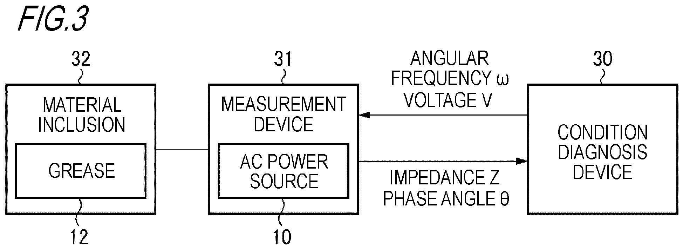

Fig. 3 is a schematic configuration diagram showing an example of the overall configuration of a system to which the condition diagnosis method according to the present embodiment can be applied. Fig. 3 shows a condition diagnosis device 30 using the condition diagnosis method according to the present embodiment, a measurement device 31, and material inclusions 32 including the grease 12 which is a target to be diagnosed. Note that the configuration shown in Fig. 1 is an example, and a different configuration may be used depending on the target to be diagnosed and the like.
The measurement device 31 includes the AC power source 10 shown in Fig. 1, and applies power to the grease 12 contained in the material inclusions 32 during diagnosis.
The condition diagnosis device 30 instructs the measurement device 31 to use the AC voltage V of the angular frequency $\omega$ of the AC power source 10 as the input value of the power to be applied to the grease 12, and acquires the impedance |Z| (|Z| indicates the absolute value of Z) of the grease 12 and the phase angle $\theta$ from the measurement device 31 as corresponding outputs (measurement values). Then, the condition diagnosis device 30 uses these values to derive parameters indicating the electrical properties of the grease 12, and then performs diagnosis. Details of parameter types and derivation methods will be described later.
The condition diagnosis device 30 may be realized by, for example, an information processing device including a control device, a storage device, and an output device (not shown). The control device may be composed of a central processing unit (CPU), a micro processing unit (MPU), a digital single processor (DSP), a dedicated circuit, or the like. The storage device is composed of volatile and non-volatile storage media such as a hard disk drive (HDD), a read only memory (ROM), and a random access memory (RAM), and it is possible to input and output various information to and from the storage device according to the instruction from the control device. The output device is composed of a speaker, a light, a display device such as a liquid crystal display, or the like, and notifies the operator according to the instruction from the control device. The output method by the output device is not particularly limited. In addition, the output device may be a network interface having a communication function, and may perform an output operation by transmitting data to an external device (not shown) via a network (not shown).
Further, the forms of the condition diagnosis device 30 and the measurement device 31 are not particularly limited. For example, the condition diagnosis device 30 and the measurement device 31 may be connected by wire or wirelessly. Alternatively, the condition diagnosis device 30 and the measurement device 31 may be integrated. Alternatively, the user may be in charge of data input/output between the condition diagnosis device 30 and the measurement device 31.

[Relative dielectric constant and relative dielectric loss factor]

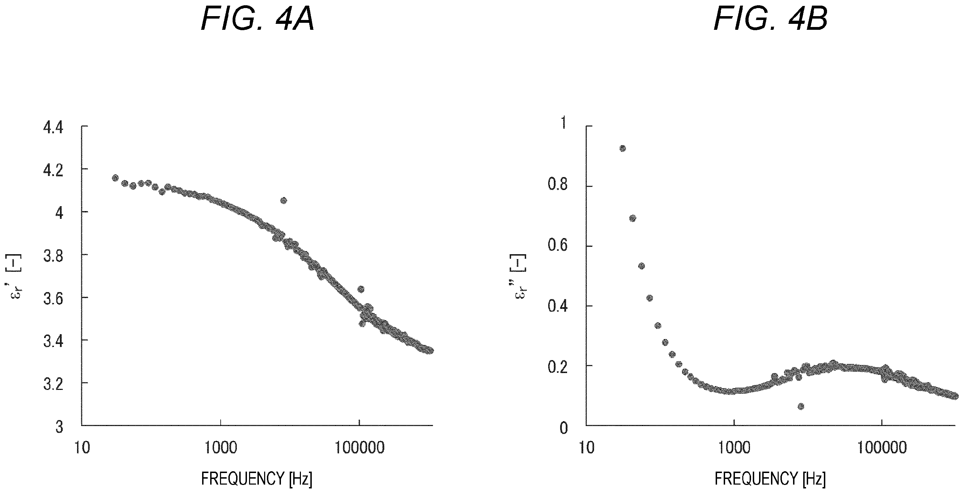

Figs. 4A and 4B are diagrams for explaining the tendency of changes in relative dielectric constant and relative dielectric loss factor according to changes in frequency. Here, as described above, the lithium 12-hydroxystearate grease will be described as an example of the grease 12. Using the configuration shown in Fig. 3, the dielectric relaxation phenomenon is confirmed by sweeping the frequency and measuring the relative dielectric constant $\varepsilon_r'$ and the relative dielectric loss factor $\varepsilon_r''$ of the grease 12.

In Fig. 4A, the horizontal axis indicates the frequency [Hz], and the vertical axis indicates the relative dielectric constant $\varepsilon_r'$. Fig. 4A shows experimental values obtained from the grease 12. As shown in Fig. 4A, the relative dielectric constant $\varepsilon_r'$ tends to decrease (monotonically decrease) as the frequency increases.

In Fig. 4B, the horizontal axis indicates the frequency [Hz], and the vertical axis indicates the relative dielectric loss factor $\varepsilon_r''$. Fig. 4B shows experimental values obtained from the grease 12. As shown in Fig. 4B, the relative dielectric loss factor $\varepsilon_r''$ once decreases as the frequency increases, then tends to increase and then decrease again.

In the present embodiment, the following seven parameters are to be derived as parameters indicating the electrical properties of the grease 12.

$\varepsilon_{r0}$: Relative dielectric constant at low-frequency limit
$\varepsilon_{r\infty}$: Relative dielectric constant at high-frequency limit
$\varepsilon_{r0} - \varepsilon_{r\infty}$: Relaxation strength
$\varepsilon_r'^-$: Average dielectric constant
$\tau$: Relaxation time [s]
$\beta$: Constant indicating distribution of relaxation time
$\sigma_0$: DC conductivity [S/m]

Although the details will be described later, the above parameters change depending on the internal condition of the grease 12. Therefore, by deriving the parameters, it is possible to use the parameters for diagnosing the internal condition of the grease.

[Fitting to theoretical formula]

The electrical properties of the grease 12 based on the dielectric relaxation phenomenon have a tendency to change as shown in Figs. 4A and 4B. In order to specify this change tendency, various parameters are derived by fitting to various theoretical formulas. Three theoretical formulas will be described here as examples, but the present invention is not limited to these. For example, other theoretical formulas may be used as long as each of the above seven parameters can be derived more accurately. Unless otherwise specified, when the same symbols are shown for the symbols used in each theoretical formula, this means that the symbols are indicating the same thing.

(Debye type)

The fitting to the Debye type theoretical formula will be described. The Debye type theoretical formula is shown below.
[Math 1]

$$\varepsilon_r' = \frac{\varepsilon_{r0} - \varepsilon_{r\infty}}{1 + \omega^2 \tau^2} + \varepsilon_{r\infty} \quad \cdots (4)$$

[Math 2]

$$\varepsilon_r'' = \frac{(\varepsilon_{r0} - \varepsilon_{r\infty})\omega\tau}{1 + \omega^2 \tau^2} \quad \cdots (5)$$

$\omega$: Angular frequency of voltage

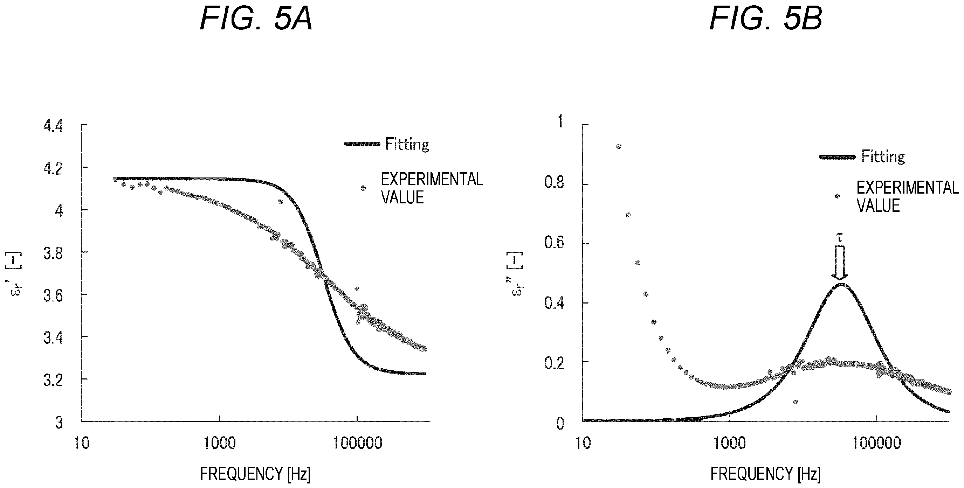

Figs. 5A and 5B compare the curve obtained by fitting to the Debye type theoretical formula and the values obtained by experiment. The experimental values in Figs. 5A and 5B are the same as those in Figs. 4A and 4B, respectively. As shown in Fig. 5B, the relaxation times $\tau$ (peak positions) match. On the other hand, non-matching results are shown for other parameters.

(Cole-Cole type)

The fitting to the Cole-Cole type theoretical formula will be described. The Cole-Cole type theoretical formula is shown below.

[Math 3]

$$\varepsilon_r{}' = \frac{1}{2}(\varepsilon_{r0} - \varepsilon_{r\infty})\left\{1 - \frac{\sinh \beta X}{\cosh \beta X + \cos\left(\frac{\beta\pi}{2}\right)}\right\} + \varepsilon_{r\infty} \quad \cdots (6)$$

[Math 4]

$$\varepsilon_r{}'' = \frac{1}{2}(\varepsilon_{r0} - \varepsilon_{r\infty})\frac{\sinh\left(\frac{\beta\pi}{2}\right)}{\cosh \beta X + \cos\left(\frac{\beta\pi}{2}\right)} \quad \cdots (7)$$

[Math 5]

$$X = \ln(\omega\tau) = \ln(2\pi f\tau) \quad \cdots (8)$$

$\pi$: Circumference ratio
f: Frequency
ln: Logarithmic function

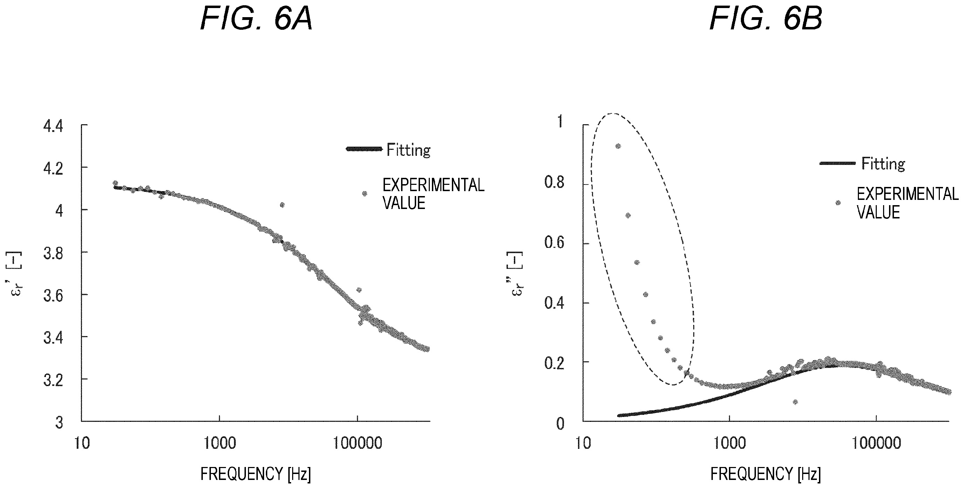

Figs. 6A and 6B compare the curve obtained by fitting to the Cole-Cole type theoretical formula and the values obtained by experiment. The experimental values in Figs. 6A and 6B are the same as those in Figs. 4A and 4B, respectively. As shown in Fig. 6A, with respect to the relative dielectric constant, the theoretical formula can express the tendency of the experimental values by fitting. On the other hand, referring to Fig. 6B, the relaxation times $\tau$ (peak position) match, but as indicated by the dashed line, the result of non-matching between the theoretical and experimental values in the tendency of relative dielectric loss factor in the low frequency range is shown.

(Cole-Cole improved type)

The fitting to the Cole-Cole improved type theoretical formula based on the Cole-Cole type will be described. The Cole-Cole improved type theoretical formula is shown below. Note that Formulas (9) and (11) are the same as Formulas (6) and (8) shown in the Cole-Cole type.

[Math 6]

$$\varepsilon_r{}' = \frac{1}{2}(\varepsilon_{r0} - \varepsilon_{r\infty})\left\{1 - \frac{\sinh \beta X}{\cosh \beta X + \cos\left(\frac{\beta\pi}{2}\right)}\right\} + \varepsilon_{r\infty} \quad \cdots (9)$$

[Math 7]

$$\varepsilon_r{}'' = \frac{\sigma_0}{2\pi f\varepsilon_0} + \frac{1}{2}(\varepsilon_{r0} - \varepsilon_{r\infty})\frac{\sinh\left(\frac{\beta\pi}{2}\right)}{\cosh \beta X + \cos\left(\frac{\beta\pi}{2}\right)} \quad \cdots (10)$$

$\varepsilon_0$: Dielectric constant of vacuum
[Math 8]

$$X = \ln(\omega\tau) = \ln(2\pi f\tau) \quad \cdots (11)$$

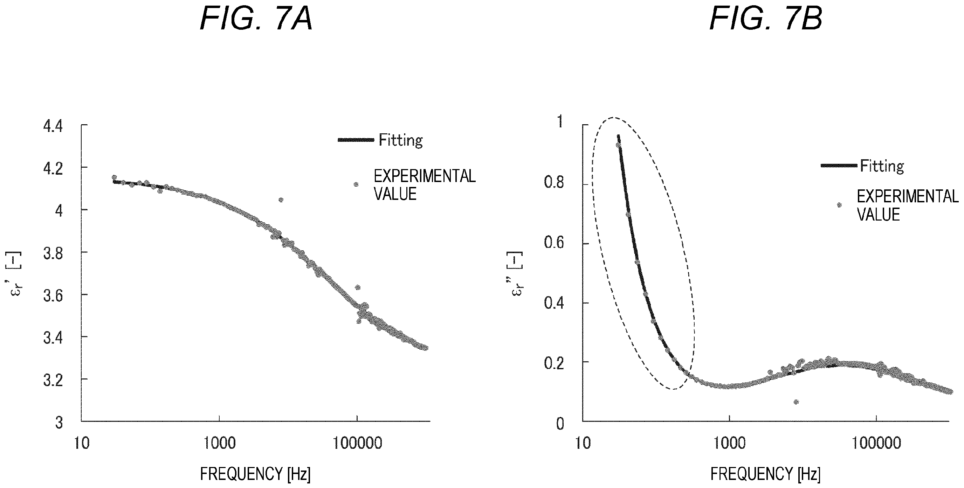

Figs. 7A and 7B compare the curve obtained by fitting to the Cole-Cole improved type theoretical formula and the values obtained by experiment. The experimental values in Figs. 7A and 7B are the same as those in Figs. 4A and 4B, respectively. As shown in Fig. 7A, with respect to the relative dielectric constant, the theoretical formula can express the tendency of the experimental values by fitting, similar to the Cole-Cole type. Further, referring to Fig. 7B, in addition to the relaxation time $\tau$ (peak position), with respect to the tendency of the relative dielectric loss factor in the low frequency range, the theoretical values can express the tendency of the experimental values by fitting.

By performing fitting to the above theoretical formula, as the parameters of the electrical properties of the grease 12, it is possible to derive the relative dielectric constant at the low-frequency limit and high-frequency limit, the relaxation strength, the average dielectric constant, the relaxation time, the distribution of relaxation time, and the DC conductivity.

[Processing flow]

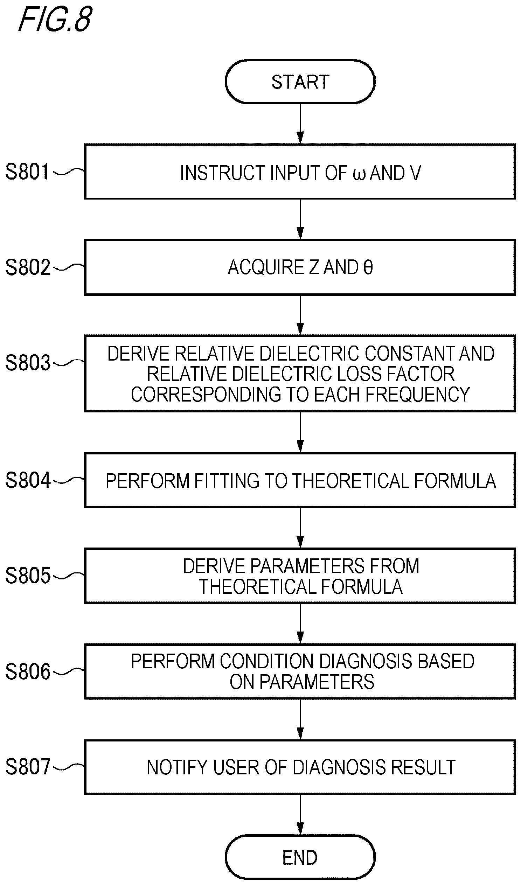

Fig. 8 is a flowchart of condition diagnosis processing according to the present embodiment. This processing is executed by the condition diagnosis device 30. For example, the control device (not shown) included in the condition diagnosis device 30 may be realized by reading a program from a storage device (not shown) for realizing the processing according to the present embodiment and executing the program.

In S801, the condition diagnosis device 30 controls the measurement device 31 to apply power of the AC voltage V with the angular frequency $\omega$ to the grease 12 using the AC power source 10 included in the measurement device 31. As a result, the AC voltage V having the angular frequency $\omega$ is applied to the grease 12.

In S802, the condition diagnosis device 30 acquires the impedance $|Z|$ and the phase angle $\theta$ from the measurement device 31 as an output for the input instructed in S801. That is, the measurement device 31 outputs the impedance $|Z|$ and the phase angle $\theta$ to the condition diagnosis device 30 as the measurement results of the grease 12 with respect to the input AC voltage V with the angular frequency $\omega$.

In S803, the condition diagnosis device 30 derives the relative dielectric constant and the relative dielectric loss factor corresponding to each frequency based on the information on the impedance $|Z|$ and the phase angle $\theta$ obtained in S802, and the information of the AC voltage V with the angular frequency $\omega$ instructed in S801. A known method may be used for the derivation method here. Further, the relative dielectric constant and the relative dielectric loss factor are derived by the measurement device 31, and the relative dielectric constant and the relative dielectric loss factor may be output to the condition diagnosis device 30 as the measurement results together with the impedance $|Z|$ and the phase angle $\theta$.

In S804, the condition diagnosis device 30 fits the obtained measurement result to the above-described theoretical formula. For example, the condition diagnosis device 30 performs fitting to the Cole-Cole improved type theoretical formulas shown in Formulas (9) to (11).

In S805, the condition diagnosis device 30 derives various parameters from the result of fitting in S804. Note that it is not necessary to derive all of the seven parameters described above at the same time, and for example, only necessary parameters may be derived according to the item which is a target to be diagnosed. The necessary parameters here may be set in any manner by the user who makes the diagnosis. In addition, an example of the relationship between each parameter and diagnostic items will be described later.

In S806, the condition diagnosis device 30 diagnoses the condition of the grease 12 based on each of the parameters derived in S805. Although the content of diagnosis here is not particularly limited, for example, a threshold value may be set for each parameter, and normality or abnormality may be diagnosed by comparison with the threshold value. Alternatively, a plurality of threshold values may be set according to the degree of urgency of the abnormality, and the degree of urgency may be diagnosed by comparing with these threshold values.

In S807, the condition diagnosis device 30 notifies the user of the diagnosis result obtained in S806. Although the notification method here is not particularly limited, for example, the parameters or items determined to be abnormal may be displayed on the screen or notified by voice. Then, this processing flow ends.

[Relationship between various parameters and condition of lubricant]

The relationship between the various parameters derived by the above-described method and the condition of the lubricant (the grease 12 in this case) will be described below.

(Relationship between amount of thickener and parameters)

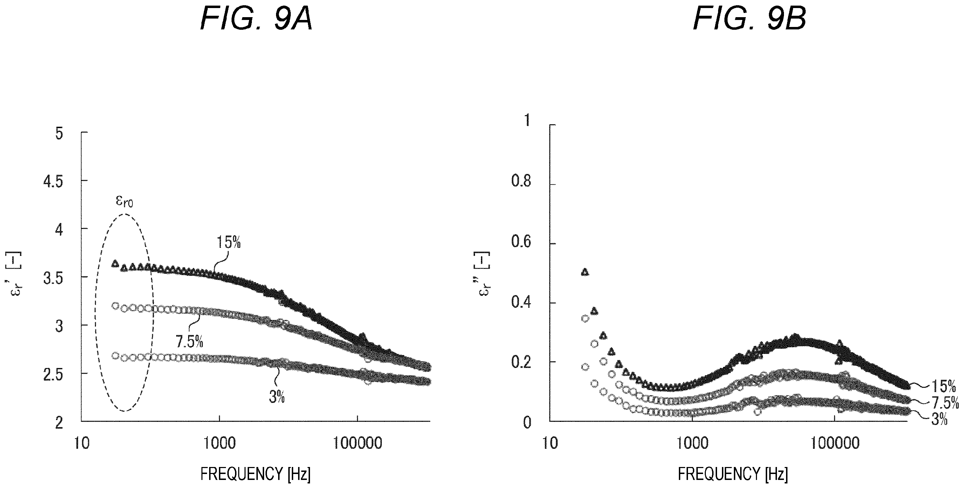

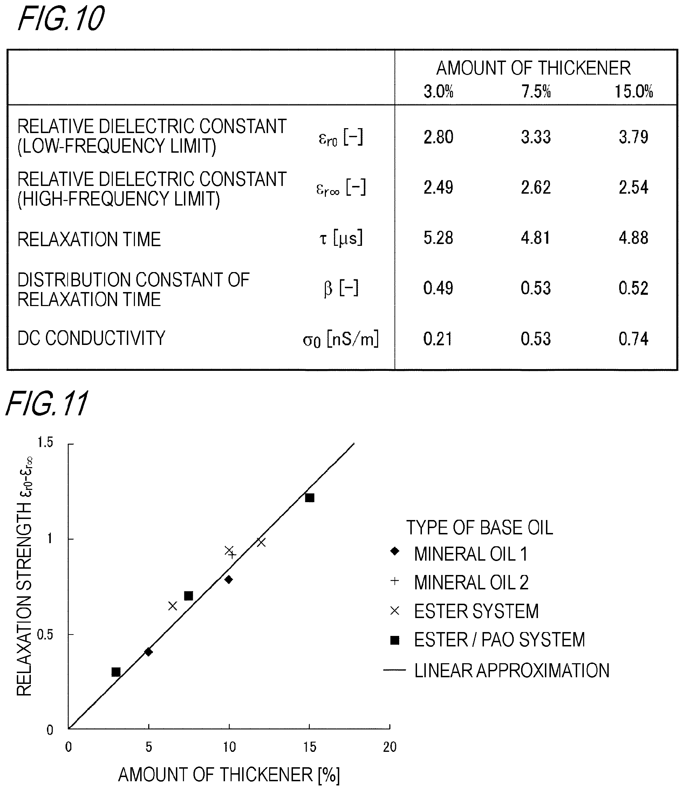

In Figs. 9A, 9B and 10, the relationship between the amount of thickener in the grease 12 and the parameters will be described. Here, an example of grease with the following composition is shown. In addition, the amount of the three thickeners (proportion [%] in grease) is shown as an example.

Base oil: ester + mineral oil
Thickener: 12OH (lithium 12-hydroxystearate grease: 12-OHStLi) (short fiber)
Amount of thickener: 3%, 7.5%, 15%

In Fig. 9A, the horizontal axis indicates the frequency [Hz], and the vertical axis indicates the relative dielectric constant $\varepsilon_r$'. As shown in Fig. 9A, the relative dielectric constant tends to decrease (monotonically decrease) as the frequency increases regardless of the amount of thickener. This tendency is similar to that described with reference to Fig. 4A. On the other hand, the relative dielectric constant $\varepsilon_{r0}$ at the low-frequency limit differs depending on the amount of thickener. Furthermore, the degree of change in relative dielectric constant with change in frequency differs depending on the amount of thickener. On the other hand, in the high frequency range, the fluctuation in the amount of thickener has little effect on the relative dielectric constant.

Since the grease 12 contains the thickener, an electric field opposite to the external electric field applied to the grease 12 is generated in the low frequency region. As a result, in the low frequency region, the dielectric constant (the relative dielectric constant $\varepsilon_{r0}$ at the low-frequency limit) increases according to the amount of thickener. On the other hand, in the high frequency region, an electric field opposite to the external electric field applied to the grease 12 is not generated. Therefore, in the high frequency region, the dielectric constant (the relative dielectric constant $\varepsilon_{r\infty}$ at the high-frequency limit) fluctuates little depending on the amount of the thickener. For this reason, a difference occurs in the relative dielectric constant $\varepsilon_{r0}$ at the low-frequency limit according to changes in amount of thickener, as shown in Fig. 9A.

In Fig. 9B, the horizontal axis indicates the frequency [Hz], and the vertical axis indicates the relative dielectric loss factor $\varepsilon_r$". As shown in Fig. 9B, the relative dielectric loss factor $\varepsilon_r$" once decreases as the frequency increases, then tends to increase and then decrease again. This tendency is similar to that described with reference to Fig. 4B.

Fig. 10 summarizes the parameters derived from the values shown in Fig. 9. As shown in Fig. 10, it can be seen that various parameters fluctuate as the amount of the thickener fluctuates. In other words, it can be understood that there is a correlation between the amount of thickener and the parameters indicating the electrical properties. Therefore, the change in the amount of thickener can be specified by referring to the parameters (in particular, the relative dielectric constant $\varepsilon_{r0}$ at the low-frequency limit) derived in the present embodiment.

Fig. 11 is a diagram for explaining the relationship between the type of base oil, the amount of thickener, and the relaxation strength. In Fig. 11, the horizontal axis indicates the amount of thickener (proportion in grease) [%], and the vertical axis indicates the relaxation strength ($\varepsilon_{r0}$ - $\varepsilon_{r\infty}$). In addition, two types of mineral oil-based, ester-based, and ester/PAO-based base oils are shown as examples. As indicated by the straight line in Fig. 11, regardless of the type of base oil, the relaxation strength increases as the amount of thickener increases. Therefore, regardless of the type of base oil, the amount of thickener can be specified by referring to the relaxation strength.

(Relationship between fiber state of thickener and parameters)

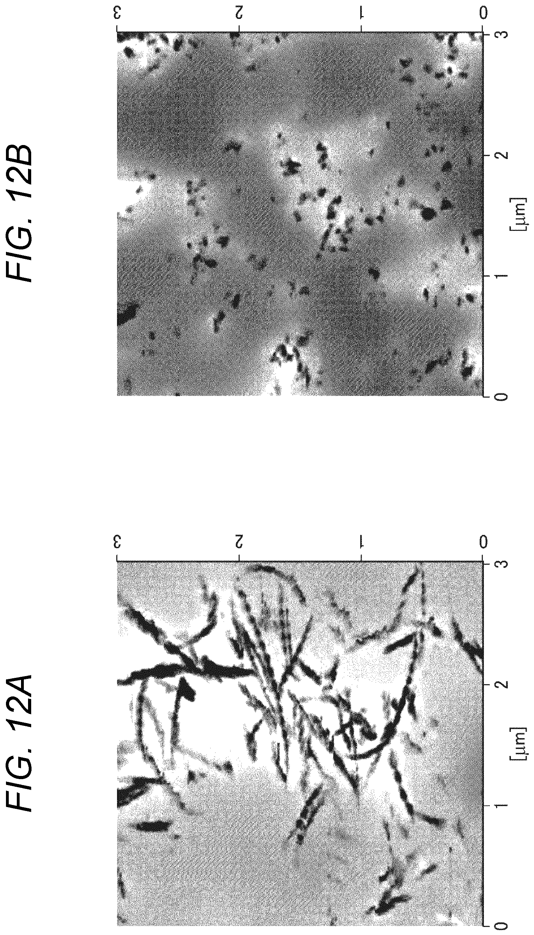

In Figs. 12A to 14, the relationship between the fiber state of thickener in the grease 12 and the parameters will be described.

Fig. 12 is a diagram for explaining the fiber state of thickener contained in the grease 12. Here, a grease containing a thickener of which the fiber structure was destroyed was produced by subjecting the grease 12 to roll processing under the following conditions.

Base oil: Mineral oil
Thickener: 12-OHStLi
Roll pressure: 1 [MPa]
Number of rolls: 5 times

Fig. 12A shows an example of the condition before roll processing, in which the fiber structure is maintained. Fig. 12B shows an example of the condition after roll processing, in which the fiber structure is destroyed. In Figs. 12A and 12B, the unit of scale is $\mu$m. In addition, the fiber state here is an example, and is not limited to this.

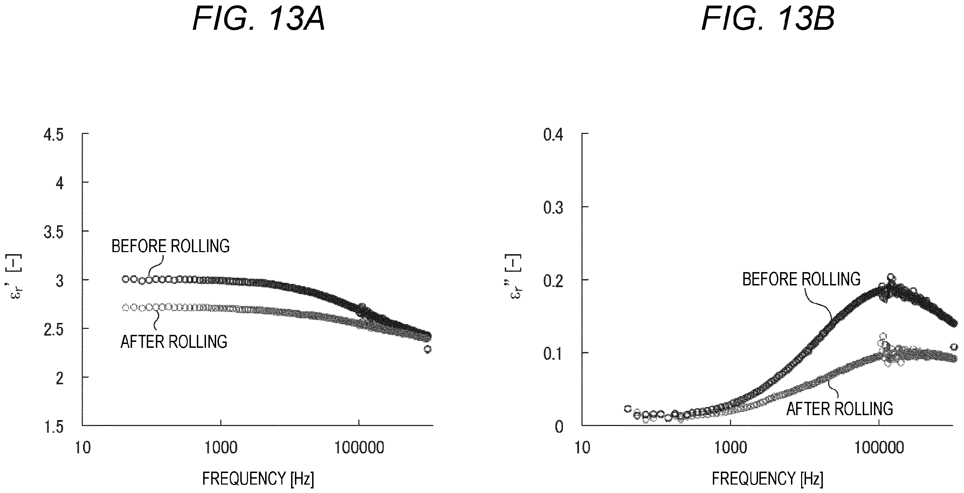

In Fig. 13A, the horizontal axis indicates the frequency [Hz], and the vertical axis indicates the relative dielectric constant $\varepsilon_r$'. As shown in Fig. 13A, the relative dielectric constant tends to decrease (monotonically decrease) as the frequency increases regardless of the fiber state of the thickener. This tendency is similar to that described with reference to Fig.

4A. On the other hand, the relative dielectric constant $\varepsilon_{r0}$ at the low-frequency limit differs depending on the fiber state of thickener. Furthermore, the degree of change in relative dielectric constant with change in frequency differs depending on the fiber state of thickener. On the other hand, in the high frequency range, the fluctuation in the amount of thickener has little effect on the relative dielectric constant.

In Fig. 13B, the horizontal axis indicates the frequency [Hz], and the vertical axis indicates the relative dielectric

loss factor $\varepsilon_r$". As shown in Fig. 13B, the relative dielectric loss factor increases once as the frequency increases, and then tends to decrease. This tendency is similar to that described with reference to Fig. 4B. In the low frequency region, the difference due to the fiber state of thickener is small, but there is a difference in the peak and distribution of the relative dielectric loss factor and the position of the peak (relaxation time $\tau$). For example, the constant $\beta$, which represents the distribution of relaxation time, tends to decrease when the fiber structure is destroyed.

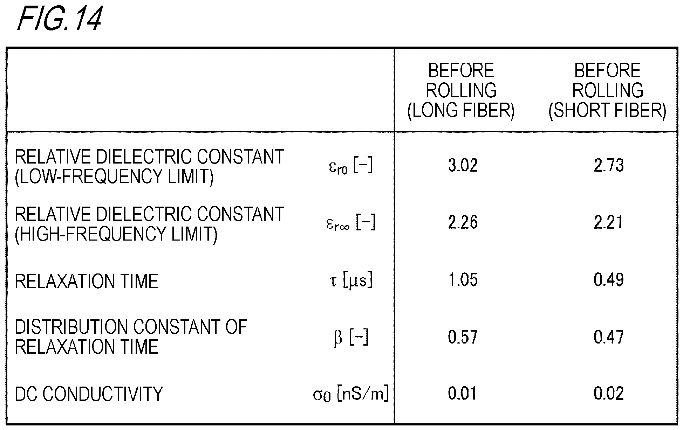

Fig. 14 summarizes the parameters derived from the values shown in Figs. 13A and 13B. As shown in Fig. 14, it can be seen that various parameters fluctuate as the fiber state of the thickener fluctuates. In other words, it can be understood that there is a correlation between the fiber state of thickener and the parameters indicating the electrical properties. Therefore, by referring to the parameters derived in the present embodiment (especially the relaxation time and the constant indicating the distribution of the relaxation time), it is possible to specify the change in the fiber state of thickener.

(Relationship between grease deterioration and parameters)

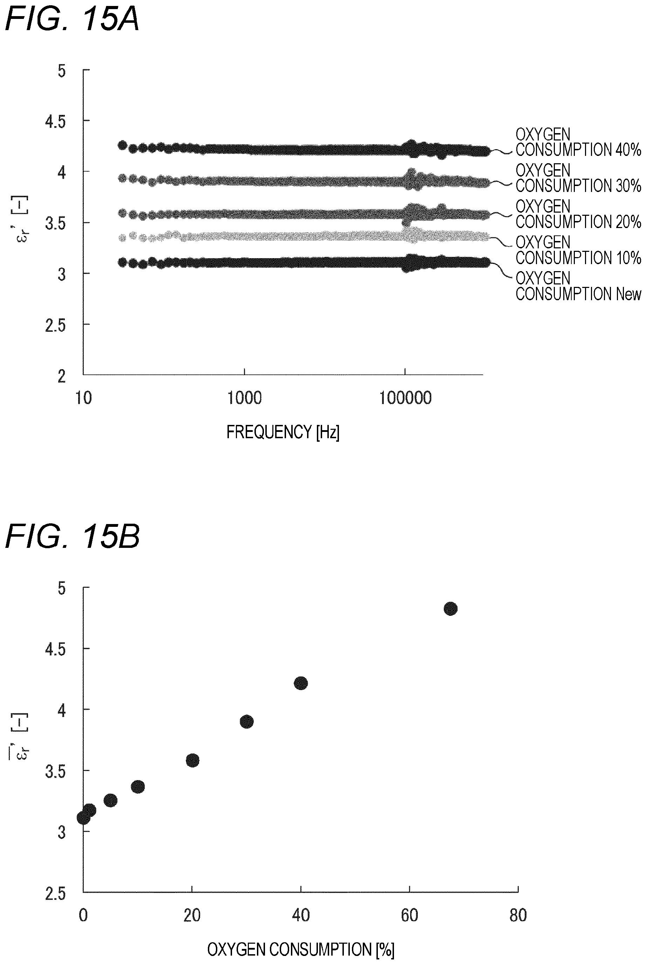

In Figs. 15A to 17B, the relationship between deterioration (oxidative deterioration) of the grease 12 and the parameters will be described. Figs. 15A to 16B are diagrams for explaining the relationship between the frequency and the deterioration (oxidative deterioration, that is, increase in oxygen consumption) degree of the base oil that forms the grease 12.

In Fig. 15A, the horizontal axis indicates the frequency [Hz], and the vertical axis indicates the relative dielectric constant $\varepsilon_r$'. As shown in Fig. 15A, the relative dielectric constant is almost constant regardless of the changes in frequency. In addition, the relative dielectric constant increases as the oxygen consumption increases.

In Fig. 15B, the horizontal axis indicates the oxygen consumption [%], and the vertical axis indicates the average dielectric constant $\varepsilon_r$'-. As shown in Fig. 15B, the average dielectric constant increases as the oxygen consumption increases.

The average dielectric constant increases as the polarity of the molecules in the base oil increases. The base oil is conventionally non-polar (for example, in a new condition), but becomes more polar due to oxidative deterioration. Therefore, the average dielectric constant increases due to oxidative deterioration of the base oil.

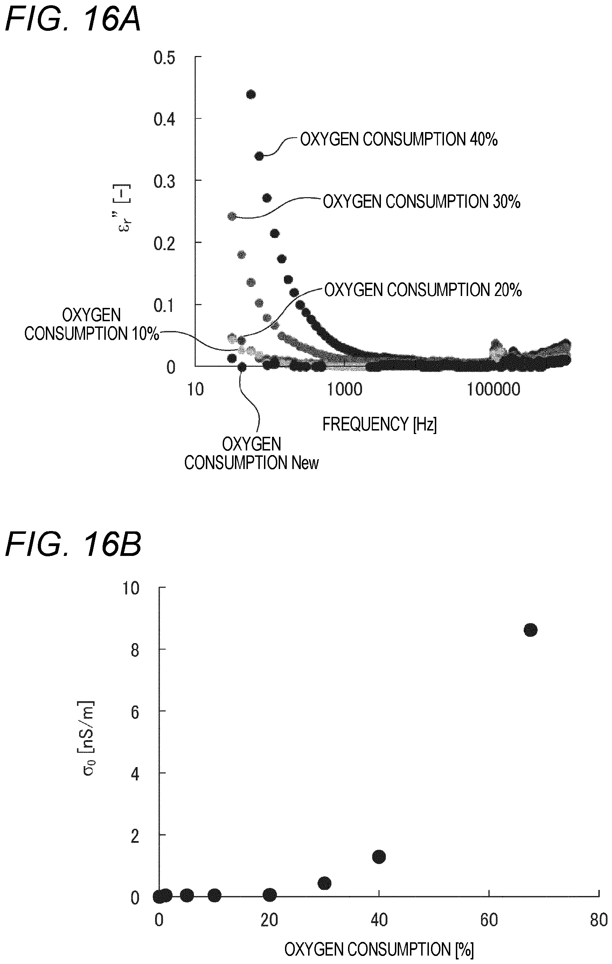

In Fig. 16A, the horizontal axis indicates the frequency [Hz], and the vertical axis indicates the relative dielectric loss factor $\varepsilon_r$". As shown in Fig. 16A, the relative dielectric loss factor in the low frequency range increases as the oxygen consumption increases.

In Fig. 16B, the horizontal axis indicates the oxygen consumption [%], and the vertical axis indicates the DC conductivity $\sigma_0$. As shown in Fig. 16B, the DC conductivity increases as the oxygen consumption increases.

From the above, it can be understood that there is a correlation between the deteriorated condition of the base oil that forms the grease 12 and the parameters indicating the electrical properties. Therefore, the deterioration degree of the base oil can be specified by referring to changes in various parameters (in particular, the average dielectric constant and the DC conductivity) derived in the present embodiment.

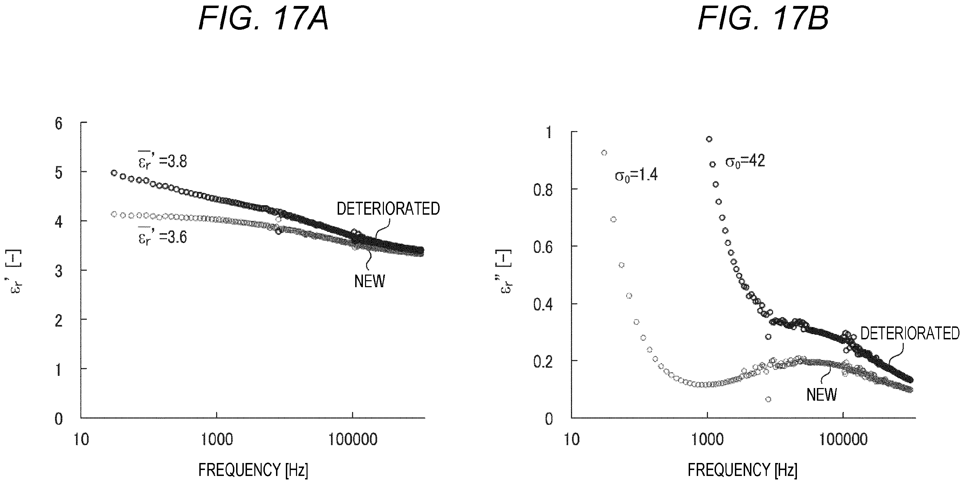

Figs. 17A and 17B are diagrams for explaining the relationship between the frequency and the relative dielectric constant and relative dielectric loss factor due to deterioration of grease. Shown here are the results of comparison between new grease and grease deteriorated by an oxidation stability tester. In Fig. 17A, the horizontal axis indicates the frequency [Hz], and the vertical axis indicates the relative dielectric constant $\varepsilon_r$'. Both new grease and deteriorated grease tend to decrease in relative dielectric constant as the frequency increases. This tendency is similar to that described with reference to Fig. 4A. On the other hand, when comparing the average dielectric constants, the deteriorated grease had a higher value. Here, the average dielectric constant of new grease was 3.6, and the average dielectric constant of deteriorated grease was 3.8.

In Fig. 17B, the horizontal axis indicates the frequency [Hz], and the vertical axis indicates the relative dielectric loss factor. In this case, when the DC conductivity $\sigma_0$ was compared, the deteriorated grease had a higher value. Here, the DC conductivity of the new grease was 1.4, and the DC conductivity of the deteriorated grease was 42.

From the above, it can be understood that there is a correlation between the deteriorated condition of the grease 12 and the parameters indicating the electrical properties. Therefore, the deterioration degree of the grease 12 can be specified by referring to changes in various parameters (in particular, the average dielectric constant and the DC conductivity) derived in the present embodiment.

(Relationship between water content and parameters)

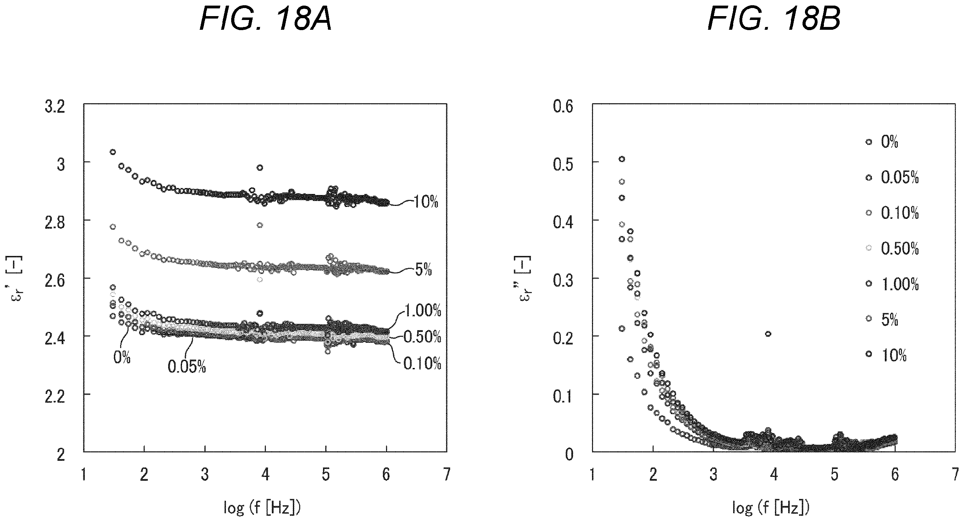

In Figs. 18A to 19, the relationship between the water content in the grease 12 and the parameters will be described. Figs. 18A and 18B is a diagram for explaining the relationship between the water content in the grease 12 and the relative dielectric constant $\varepsilon_r$' and the relative dielectric loss factor $\varepsilon_r$". Here, an example in which the measurement is performed while the grease 12 contains 0 to 10% moisture, the voltage is 1.0 V, and the frequency is in the range of 30 Hz to 1 MHz will be described.

In Fig. 18A, the horizontal axis indicates the logarithm of the frequency [Hz], and the vertical axis indicates the relative dielectric constant $\varepsilon_r$'. As shown in Fig. 18A, the relative dielectric constant $\varepsilon_r$' tends to show a substantially constant value depending on the water content regardless of the changes in frequency. In addition, the relative dielectric constant $\varepsilon_r$' tends to show a higher value as the water content in the grease 12 increases.

In Fig. 18B, the horizontal axis indicates the logarithm of the frequency [Hz], and the vertical axis indicates the relative dielectric loss factor $\varepsilon_r$". As shown in Fig. 18B, even when the water content in the grease 12 increases, there is no significant difference in the change tendency of the relative dielectric loss factor. In other words, regardless of the water content, the change tendency of the relative dielectric loss factor $\varepsilon_r$" when the frequency fluctuates is almost the same.

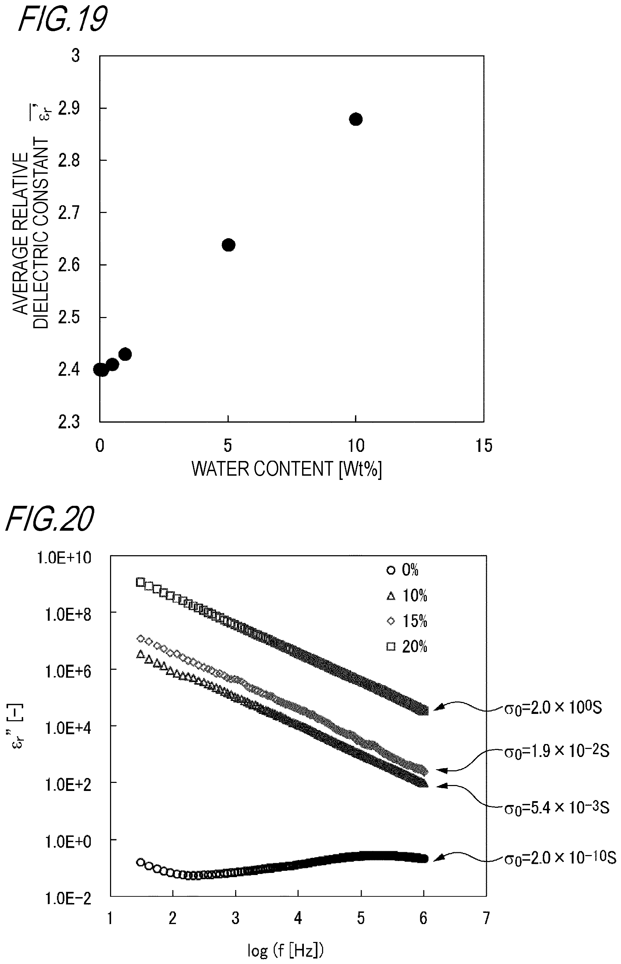

Fig. 19 is a diagram for explaining the relationship between the water content in the grease 12 and the average dielectric constant $\varepsilon_r$'-. In Fig. 19, the horizontal axis indicates the water content [% wt] in the grease 12, and the vertical axis indicates the average dielectric constant $\varepsilon_r$'-. Here, the grease 12 is completely mixed with water. As shown in Fig. 19, the average dielectric constant $\varepsilon_r$'- tends to increase (monotonically increase) as the water content increases.

In view of the above, it can be understood that there is a correlation between the water content in the grease 12 and the parameters indicating electrical properties, specifically the correlation between the relative dielectric constant $\varepsilon_r$' and the average dielectric constant $\varepsilon_r$'-. Therefore, by paying attention to these parameters, it is possible to specify the contamination of the grease 12 with moisture. The deterioration of the grease 12 has been described with reference to Figs. 15A to 17B, but the change tendency of the parameters at this time (for example, the average dielectric constant $\varepsilon_r$'- of Fig. 15B and the DC conductivity $\sigma_0$ of Fig. 16B) and the change tendency of the parameters shown in Figs. 18A and 18B or 19 are different. Therefore, by capturing the difference in these changes, it is possible to separately detect the deterioration of the grease and the contamination of the grease with water.

(Relationship between iron powder amount and parameters)

In Fig. 20, the relationship between the iron powder amount in the grease 12 and the parameters will be described. Fig. 20 is a diagram for explaining the relationship between the iron powder amount in the grease 12 and the relative dielectric loss factor $\varepsilon_r$". Due to the presence of iron powder in the grease 12, the DC conductivity $\sigma_0$ extremely increases. While the change in DC conductivity due to other factors is on a scale of approximately 10 to 100 times, the increase in DC conductivity due to the contamination (increase) of iron powder shows a change of $10^6$ or more. Here, detection is performed focusing on the change. Here, an example in which the measurement is performed while the grease 12 contains 0 to 20% moisture, the voltage is 1.0 V, and the frequency is in the range of 30 Hz to 1 MHz will be described.

In Fig. 20, the horizontal axis indicates the logarithm of the frequency [Hz], and the vertical axis indicates the relative dielectric loss factor $\varepsilon_r$". Here, four examples are shown in which the iron powder amount contained in the grease 12 (weight ratio to the grease) is 0%, 10%, 15%, and 20%. When the iron powder amount is 0%, 10%, 15%, and 20%, the DC conductivity $\sigma_0$ is $2.0 \times 10^{-10}$S, $5.4 \times 10^{-3}$S, $1.9 \times 10^{-2}$S, and $2.0 \times 10$S. In other words, the value of the DC conductivity $\sigma_0$ is extremely different depending on the iron powder amount in the grease 12. Therefore, by evaluating the value of the DC conductivity $\sigma_0$, it is possible to measure the iron powder amount in the grease 12, that is, the contamination of iron powder.

[Summary]

In the present embodiment, parameters related to dielectric relaxation are derived as electrical properties for specifying the condition of a lubricant (grease in the present example). Each parameter can be used when specifying the condition of the lubricant as follows.

Relative dielectric constant ($\varepsilon_{r0}$) at low-frequency limit: amount of thickener

Relative dielectric constant ($\varepsilon_{r\infty}$) at high-frequency limit: type of base oil
Relaxation strength ($\varepsilon_{r0}$ - $\varepsilon_{r\infty}$): amount of thickener
Average dielectric constant ($\varepsilon_r$'-): deterioration degree of base oil, water content in grease
Relaxation time ($\tau$): fiber state of thickener, deterioration degree of grease
Constant ($\beta$) that indicates distribution of relaxation time: fiber state of thickener, deterioration degree of grease
DC conductivity ($\sigma_0$): amount of thickener, deterioration degree of base oil, deterioration degree of grease, amount of additives, iron powder amount in grease

Note that the correlation between each parameter and the condition of the lubricant is an example, and is not limited to the above. For example, one condition item may be diagnosed from a plurality of parameters, or a plurality of condition items may be diagnosed from one parameter. Further, the condition may be diagnosed after specifying the correlation between the parameters and the condition according to the composition that forms the lubricant.

As described above, according to the present embodiment, it is possible to specify the parameters indicating the electrical properties of the lubricant without destroying the target to be diagnosed. Then, it is possible to easily diagnose the condition of the lubricant based on the parameters indicating the electrical properties.

<Other embodiments>

In addition, in the present invention, a program or application for realizing the functions of one or more embodiments described above is supplied to a system or device using a network or a storage medium, and can be realized by processing executed as one or more processors in the computer of the system or device reads the program.

Further, the circuit that realizes one or more functions may realize an application specific integrated circuit (ASIC) or a field programmable gate array (FPGA).

As described above, the present invention is not limited to the above-described embodiments, it is also possible for those skilled in the art to combine each configuration of the embodiments with each other, modify and apply based on the description of the specification and well-known technology, and the combinations, modifications, and applications are within the scope for which protection is sought.

As described above, this specification discloses the following matters.

(1) A condition diagnosis method including: a measurement step of measuring relative dielectric constant of a lubricant by applying a voltage to the lubricant while changing a frequency from an AC power source; a derivation step of deriving parameters indicating electrical properties of the lubricant by applying the relative dielectric constant measured in the measurement step to a theoretical formula; and a diagnosis step of diagnosing a condition of the lubricant using the parameters.

According to this configuration, it is possible to easily diagnose the condition of a lubricant without destroying the target to be diagnosed.

(2) The condition diagnosis method according to (1), in which the parameters include at least one of relative dielectric constant at low-frequency limit, relative dielectric constant at high-frequency limit, relaxation strength, average dielectric constant, relaxation time, distribution of relaxation time, and DC conductivity.

According to this configuration, it is possible to derive a plurality of parameters for diagnosing the condition of the lubricant.

(3) The condition diagnosis method according to (1) or (2), in which the theoretical formulas are expressed by

[Math 9],

$$\varepsilon_r{}' = \frac{1}{2}(\varepsilon_{r0} - \varepsilon_{r\infty})\left\{1 - \frac{\sinh\beta X}{\cosh\beta X + \cos\left(\frac{\beta\pi}{2}\right)}\right\} + \varepsilon_{r\infty}$$

[Math 10], and

$$\varepsilon_r{}'' = \frac{\sigma_0}{2\pi f \varepsilon_0} + \frac{1}{2}\left(\varepsilon_{r0} - \varepsilon_{r\infty}\right)\frac{\sinh\left(\frac{\beta\pi}{2}\right)}{\cosh\beta X + \cos\left(\frac{\beta\pi}{2}\right)}$$

[Math 11].

$$X = \ln(\omega\tau) = \ln(2\pi f \tau)$$

According to this configuration, it is possible to derive the theoretical values of the relative dielectric constant and the relative dielectric loss factor with higher accuracy.

(4) The condition diagnosis method according to any one of (1) to (3), in which the lubricant is in a bulk state.

According to this configuration, it is possible to diagnose the condition of the lubricant in the bulk state.

(5) The condition diagnosis method according to any one of (1) to (4), in which the lubricant is lubricating oil.

According to this configuration, it is possible to treat lubricating oil as a target to be diagnosed.

**EP 4 224 160 A1**

(6) The condition diagnosis method according to any one of (1) to (4), in which the lubricant is grease.

According to this configuration, grease can be treated as a target to be diagnosed.

(7) The condition diagnosis method according to (6), in which, in the diagnosis step, at least one of an amount of thickener, a fiber state of thickener, a deterioration degree of grease, a water content in grease, and an iron powder amount in grease is diagnosed as a condition of the grease.

According to this configuration, it is possible to diagnose a plurality of conditions of grease.

(8) A condition diagnosis device including: a measurement unit for measuring relative dielectric constant of a lubricant by applying a voltage to the lubricant while changing a frequency from an AC power source; a derivation unit for deriving parameters indicating electrical properties of the lubricant by applying the relative dielectric constant measured in the measurement unit to a theoretical formula; and a diagnosis unit for diagnosing a condition of the lubricant using the parameters.

According to this configuration, it is possible to easily diagnose the condition of a lubricant without destroying the target to be diagnosed.

(9) A program for causing a computer to execute a measurement step of measuring relative dielectric constant of a lubricant by applying a voltage to the lubricant while changing a frequency from an AC power source, a derivation step of deriving parameters indicating electrical properties of the lubricant by applying the relative dielectric constant measured in the measurement step to a theoretical formula, and a diagnosis step of diagnosing a condition of the lubricant using the parameters.

According to this configuration, it is possible to easily diagnose the condition of a lubricant without destroying the target to be diagnosed.

Although various embodiments have been described above with reference to the drawings, it is needless to say that the present invention is not limited to such examples. In a case of those skilled in the art, it is apparent that various modification examples or correction examples can be assumed within the scope described in the claims, and it is understood that the modification examples or correction examples belong to the technical scope of the present invention. Moreover, each component in the above embodiments may be combined in any manner without departing from the gist of the invention.

This application is based on a Japanese patent application filed on September 29, 2020 (Japanese patent application 2020-163961) and a Japanese patent application filed on August 25, 2021 (Japanese patent application 2021-137562), the contents of which are incorporated by reference into this application.

REFERENCE SIGNS LIST

10    AC power source
11    Electrode
12    Grease
30    Condition diagnosis device
31    Measurement device
32    Material inclusion

**Claims**

1. A condition diagnosis method comprising:

   a measurement step of measuring relative dielectric constant of a lubricant by applying a voltage to the lubricant while changing a frequency from an AC power source;
   a derivation step of deriving parameters indicating electrical properties of the lubricant by applying the relative dielectric constant measured in the measurement step to a theoretical formula; and
   a diagnosis step of diagnosing a condition of the lubricant using the parameters.

2. The condition diagnosis method according to claim 1, wherein
   the parameters include at least one of relative dielectric constant at low-frequency limit, relative dielectric constant at high-frequency limit, relaxation strength, average dielectric constant, relaxation time, distribution of relaxation time, and DC conductivity.

3. The condition diagnosis method according to claim 1 or 2, wherein the theoretical formulas are expressed by:

[Math 1]

$$\varepsilon_r{}' = \frac{1}{2}(\varepsilon_{r0} - \varepsilon_{r\infty})\left\{1 - \frac{\sinh\beta X}{\cosh\beta X + \cos\left(\frac{\beta\pi}{2}\right)}\right\} + \varepsilon_{r\infty}$$

;

[Math 2]

$$\varepsilon_r{}'' = \frac{\sigma_0}{2\pi f\varepsilon_0} + \frac{1}{2}\left(\varepsilon_{r0} - \varepsilon_{r\infty}\right)\frac{\sinh\left(\frac{\beta\pi}{2}\right)}{\cosh\beta X + \cos\left(\frac{\beta\pi}{2}\right)}$$

;

and

[Math 3]

$$X = \ln(\omega\tau) = \ln(2\pi f\tau)$$

4. The condition diagnosis method according to any one of claims 1 to 3, wherein the lubricant is in a bulk state.

5. The condition diagnosis method according to any one of claims 1 to 4, wherein the lubricant is lubricating oil.

6. The condition diagnosis method according to any one of claims 1 to 4, wherein the lubricant is grease.

7. The condition diagnosis method according to claim 6, wherein in the diagnosis step, at least one of an amount of thickener, a fiber state of thickener, a deterioration degree of grease, a water content in grease, and an iron powder amount in grease is diagnosed as a condition of the grease.

8. A condition diagnosis device comprising:

a measurement unit for measuring relative dielectric constant of a lubricant by applying a voltage to the lubricant while changing a frequency from an AC power source;
a derivation unit for deriving parameters indicating electrical properties of the lubricant by applying the relative dielectric constant measured by the measurement unit to a theoretical formula; and
a diagnosis unit for diagnosing a condition of the lubricant using the parameters.

9. A program for causing a computer to execute:

a measurement step of measuring relative dielectric constant of a lubricant by applying a voltage to the lubricant while changing a frequency from an AC power source;
a derivation step of deriving parameters indicating electrical properties of the lubricant by applying the relative dielectric constant measured in the measurement step to a theoretical formula; and
a diagnosis step of diagnosing a condition of the lubricant using the parameters.

*FIG.1*

*FIG.2*

## FIG.3

| 32 | | 31 | ANGULAR FREQUENCY ω VOLTAGE V | 30 |
|---|---|---|---|---|
| MATERIAL INCLUSION | | MEASUREMENT DEVICE | | CONDITION DIAGNOSIS DEVICE |
| GREASE | | AC POWER SOURCE | IMPEDANCE Z PHASE ANGLE θ | |
| 12 | | 10 | | |

FIG. 4A

FIG. 4B

FIG. 5A

FIG. 5B

FIG. 6A

FIG. 6B

## FIG. 7A

## FIG. 7B

EP 4 224 160 A1

## FIG.8

```
                    ┌─────────────┐
                    │    START    │
                    └─────────────┘
                           │
                           ▼
S801 ─┐  ┌──────────────────────────────────────────┐
      └──│         INSTRUCT INPUT OF ω AND V         │
         └──────────────────────────────────────────┘
                           │
                           ▼
S802 ─┐  ┌──────────────────────────────────────────┐
      └──│             ACQUIRE Z AND θ               │
         └──────────────────────────────────────────┘
                           │
                           ▼
S803 ─┐  ┌──────────────────────────────────────────┐
      └──│  DERIVE RELATIVE DIELECTRIC CONSTANT AND  │
         │    RELATIVE DIELECTRIC LOSS FACTOR        │
         │   CORRESPONDING TO EACH FREQUENCY         │
         └──────────────────────────────────────────┘
                           │
                           ▼
S804 ─┐  ┌──────────────────────────────────────────┐
      └──│   PERFORM FITTING TO THEORETICAL FORMULA  │
         └──────────────────────────────────────────┘
                           │
                           ▼
S805 ─┐  ┌──────────────────────────────────────────┐
      └──│       DERIVE PARAMETERS FROM              │
         │       THEORETICAL FORMULA                 │
         └──────────────────────────────────────────┘
                           │
                           ▼
S806 ─┐  ┌──────────────────────────────────────────┐
      └──│  PERFORM CONDITION DIAGNOSIS BASED        │
         │          ON PARAMETERS                    │
         └──────────────────────────────────────────┘
                           │
                           ▼
S807 ─┐  ┌──────────────────────────────────────────┐
      └──│      NOTIFY USER OF DIAGNOSIS RESULT      │
         └──────────────────────────────────────────┘
                           │
                           ▼
                    ┌─────────────┐
                    │     END     │
                    └─────────────┘
```

FIG. 9B

FIG. 9A

## FIG.10

| | | AMOUNT OF THICKENER | | |
| | | 3.0% | 7.5% | 15.0% |
|---|---|---|---|---|
| RELATIVE DIELECTRIC CONSTANT (LOW-FREQUENCY LIMIT) | $\varepsilon_{r0}$ [–] | 2.80 | 3.33 | 3.79 |
| RELATIVE DIELECTRIC CONSTANT (HIGH-FREQUENCY LIMIT) | $\varepsilon_{r\infty}$ [–] | 2.49 | 2.62 | 2.54 |
| RELAXATION TIME | $\tau$ [µs] | 5.28 | 4.81 | 4.88 |
| DISTRIBUTION CONSTANT OF RELAXATION TIME | $\beta$ [–] | 0.49 | 0.53 | 0.52 |
| DC CONDUCTIVITY | $\sigma_0$ [nS/m] | 0.21 | 0.53 | 0.74 |

## FIG.11

TYPE OF BASE OIL
◆ MINERAL OIL 1
+ MINERAL OIL 2
× ESTER SYSTEM
■ ESTER / PAO SYSTEM
— LINEAR APPROXIMATION

FIG. 13A

FIG. 13B

EP 4 224 160 A1

## FIG.14

| | | BEFORE ROLLING (LONG FIBER) | BEFORE ROLLING (SHORT FIBER) |
|---|---|---|---|
| RELATIVE DIELECTRIC CONSTANT (LOW-FREQUENCY LIMIT) | $\varepsilon_{r0}$ [−] | 3.02 | 2.73 |
| RELATIVE DIELECTRIC CONSTANT (HIGH-FREQUENCY LIMIT) | $\varepsilon_{r\infty}$ [−] | 2.26 | 2.21 |
| RELAXATION TIME | $\tau$ [μs] | 1.05 | 0.49 |
| DISTRIBUTION CONSTANT OF RELAXATION TIME | $\beta$ [−] | 0.57 | 0.47 |
| DC CONDUCTIVITY | $\sigma_0$ [nS/m] | 0.01 | 0.02 |

## FIG. 15A

## FIG. 15B

## FIG. 16A

## FIG. 16B

# FIG. 17A

# FIG. 17B

EP 4 224 160 A1

FIG. 18A

FIG. 18B

EP 4 224 160 A1

*FIG.19*

*FIG.20*

<div align="center">

**INTERNATIONAL SEARCH REPORT**

</div>

| International application No. |
|---|
| **PCT/JP2021/035203** |

**A. CLASSIFICATION OF SUBJECT MATTER**

*G01N 33/30*(2006.01)i; *G01N 27/00*(2006.01)i; *G01N 27/02*(2006.01)i; *G01N 27/22*(2006.01)i
FI: G01N33/30; G01N27/00 L; G01N27/02 Z; G01N27/22 B

According to International Patent Classification (IPC) or to both national classification and IPC

**B. FIELDS SEARCHED**

Minimum documentation searched (classification system followed by classification symbols)

G01N33/30; G01N27/00; G01N27/02; G01N27/22

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| X | WO 2011/065340 A1 (IDEMITSU KOSAN CO., LTD.) 03 June 2011 (2011-06-03) claims, paragraphs [0013]-[0027], fig. 1-4 | 1, 4-5, 8-9 |
| Y | | 6-7 |
| Y | WO 2014/094813 A1 (AKTIEBOLAGET SKF) 26 June 2014 (2014-06-26) claims, paragraphs [0033]-[0055], fig. 1-4 | 6-7 |
| A | JP 2009-198341 A (MITSUBISHI HEAVY INDUSTRIES, LTD.) 03 September 2009 (2009-09-03) entire text, all drawings | 1-9 |
| A | JP 2000-338101 A (NGK SPARK PLUG CO.) 08 December 2000 (2000-12-08) entire text, all drawings | 1-9 |
| A | JP 2017-211329 A (KYB CORP.) 30 November 2017 (2017-11-30) entire text, all drawings | 1-9 |
| A | JP 2007-192769 A (NTN CORP.) 02 August 2007 (2007-08-02) entire text, all drawings | 1-9 |

☐ Further documents are listed in the continuation of Box C. ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **30 November 2021** | **14 December 2021** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

**INTERNATIONAL SEARCH REPORT**
**Information on patent family members**

International application No.

**PCT/JP2021/035203**

| Patent document cited in search report | | | Publication date (day/month/year) | Patent family member(s) | | | Publication date (day/month/year) |
|---|---|---|---|---|---|---|---|
| WO | 2011/065340 | A1 | 03 June 2011 | US | 2012/0229151 | A1 | |
| | | | | claims, paragraphs [0030]-[0090], fig. 1-4 | | | |
| | | | | EP | 2505999 | A1 | |
| | | | | CN | 102667461 | A | |
| | | | | KR | 10-2012-0098732 | A | |
| | | | | JP | 5706830 | B2 | |
| WO | 2014/094813 | A1 | 26 June 2014 | EP | 2932247 | A1 | |
| JP | 2009-198341 | A | 03 September 2009 | US | 2009/0216471 | A1 | |
| | | | | entire text, all drawings | | | |
| JP | 2000-338101 | A | 08 December 2000 | (Family: none) | | | |
| JP | 2017-211329 | A | 30 November 2017 | (Family: none) | | | |
| JP | 2007-192769 | A | 02 August 2007 | US | 2010/0157304 | A1 | |
| | | | | entire text, all drawings | | | |
| | | | | EP | 1980840 | A1 | |
| | | | | WO | 2007/083520 | A1 | |

Form PCT/ISA/210 (patent family annex) (January 2015)

32

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2008215901 A
- JP H7239316 A

- JP 2020163961 A
- JP 2021137562 A